# EUROPEAN PATENT SPECIFICATION

(11) **EP 0 777 684 B1**
(45) Date of publication and mention of the grant of the patent: **28.04.2004**
(21) Application number: 94930402.6
(22) Date of filing: 16.08.1994
(51) Int. Cl.: C07K 14/00, C07K 14/705, C07K 16/00, C12N 15/00, A61K 38/17, C07K 14/72

(54) **CALCITONIN RECEPTOR**
CALCITONINREZEPTOR
RECEPTEUR DE CALCITONINE

(43) Date of publication of application: 11.06.1997
(73) Proprietor: HUMAN GENOME SCIENCES, INC., Rockville, MD 20850-3338 (US)
(72) Inventor: LI, Yi, Gaithersburg, MD 20878 (US); LEE, Norman H., Woodstock, MD 21163 (US)
(74) Representative: VOSSIUS & PARTNER
(86) International application number: PCT/US1994/009235
(87) International publication number: WO 1996/005221

(56) References cited:
- WO-A-92/01810
- WO-A-94/08006
- FOORD S M ET AL: "ISOLATION AND CHARACTERISATION OF A HUMAN CALCITONIN-GENE-RELATED- PEPTIDE RECEPTOR" EUROPEAN JOURNAL OF BIOCHEMISTRY, vol. 170, no. 1/02, 30 December 1987, STUTTGART DE, pages 373-379, XP002041589
- WIMALAWANSA S J ET AL: "ISOLATION, PURIFICATION, AND CHARACTERIZATION OF CALCITONIN GENE-RELATED PEPTIDE RECEPTOR" PEPTIDES, vol. 14, 1993, NEW YORK US, pages 691-699, XP002062248
- MUFF R ET AL.: "COMPARISON OF A CALCITONIN GENE-RELATED PEPTIDE RECEPTOR IN A HUMAN NEUROBLASTOMA CELL LINE SK-N-MC AND A CALCITONIN RECEPTOR IN A HUMAN BREAST CARCINOMA CELL LINE T47D" ANNALS OF THE NEW YORK ACADEMY OF SCIENCES, vol. 657, 1992, NEW YORK US, pages 106-116, XP002062249
- AIYAR N ET AL: "A CDNA ENCODING THE CALCITONIN GENE-RELATED PEPTIDE TYPE 1 RECEPTOR" JOURNAL OF BIOLOGICAL CHEMISTRY, vol. 271, no. 19, 10 May 1996, BALTIMORE US, pages 11325-11329, XP002023845
- NEURON, Volume 11, issued December 1993, CHANG et al., "Identification of a Seven Transmembrane Helix Receptor for Corticotropin-Releasing Factor and Sauvagine in Mammalian Brain", pages 1187-1195.
- SCIENCE, Volume 254, issued 15 November 1991, LIN et al., "Expression Cloning of an Adenylate Cyclase-Coupled Calcitonin Receptor", pages 1022-1024.
- CLINICAL SCIENCE, Volume 85, issued 1993, NJUKI et al., "A New Calcitonin-Receptor-Like Sequence in Rat Pulmonary Blood Vessels", pages 385-388.
- THE EMBO JOURNAL, Volume 10, Number 7, issued October 1991, ISHIHARA et al., "Molecular Cloning and Expression of a cDNA Encoding the Secretin Receptor", pages 1635-1641.

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention is a human 7-transmembrane receptor. The transmembrane receptor is a G-protein coupled receptor. More particularly, the 7-transmembrane receptor has been putatively identified as a calcitonin receptor, sometimes hereinafter referred to as "CTR". The invention also relates to inhibiting the action of such polypeptides.

It is well established that many medically significant biological processes are mediated by proteins participating in signal transduction pathways that involve G-proteins and/or second messengers, e.g., cAMP (Lefkowitz, Nature, 351:353-354 (1991)). Herein these proteins are referred to as proteins participating in pathways with G-proteins or PPG proteins. Some examples of these proteins include the GPC receptors, such as those for adrenergic agents and dopamine (Kobilka, B.K., et al., PNAS, 84:46-50 (1987); Kobilka, B.K., et al., Science, 238:650-656 (1987); Bunzow, J.R., et al., Nature, 336:783-787 (1988)), G-proteins themselves, effector proteins, e.g., phospholipase C, adenyl cyclase, and phosphodiesterase, and actuator proteins, e.g., protein kinase A and protein kinase C (Simon, M.I., et al., Science, 252:802-8 (1991)).

For example, in one form of signal transduction, the effect of hormone binding is activation of an enzyme, adenylate cyclase, inside the cell. Enzyme activation by hormones is dependent on the presence of the nucleotide GTP, and GTP also influences hormone binding. A G-protein connects the hormone receptors to adenylate cyclase. G-protein was shown to exchange GTP for bound GDP when activated by hormone receptors. The GTP-carrying form then binds to an activated adenylate cyclase. Hydrolysis of GTP to GDP, catalyzed by the G-protein itself, returns the G-protein to its basal, inactive form. Thus, the G-protein serves a dual role, as an intermediate that relays the signal from receptor to effector, and as a clock that controls the duration of the signal.

Calcitonin receptors act by binding calcitonin which activates adenylyl cyclase via a G-protein. Another major pathway for intracellular signalling by calcitonin is through increases in cytosolic cAMP.

The calcitonin receptor cloned by Lin et al., Science, 254:1022-1024, (1991), is thought to couple to G_{Sα}, the heterotrimeric guanosine triphosphate (GTP)-binding protein that is sensitive to cholera toxin. This receptor can also couple to an additional signalling pathway via a pertussis toxin-sensitive G-protein in isolated osteoclasts.

The recombinant calcitonin receptor described in Chabre et al., Molecular Endocrinology, 6:551-556 (1992) is functionally coupled to increases in intracellular cAMP, presumably by coupling to G_{S}. Thus there seems to be a new family of G-protein-coupled receptors that activate adenylyl cyclase.

The calcitonin receptors bind to calcitonin which is a peptide hormone of 32 amino acids that was initially identified as a hypocalcemic factor secreted by the parafollicular cells of the thyroid gland in response to elevations in serum calcium (Copp et al., Endocrinology, 70:638-649 (1962). The hypocalcemic effect of calcitonin is mediated primarily by direct inhibition of osteoclast-mediated bone resorption (Freidman and Raisz, Science, 150:1465-1467, (1965)). Calcitonin also enhances renal calcium excretion (Haas et al., J. Clin. Invest., 50:2689-2701 (1972)). In addition to receptors in bone and kidney, high affinity calcitonin binding sites have been demonstrated in many different tissues including the central nervous system, testes, placenta, lung and on spermatozoa. Cells derived from lung and breast carcinomas, as well as certain lymphoid and myeloid cell lines also express receptors for this hormone. Although this physiologic role of calcitonin in many of these tissue remains poorly understood, its action clearly extends beyond its effect on calcium homeostasis.

In accordance with one aspect of the present invention, there are provided novel polypeptides which have been putatively identified as a calcitonin receptor, as well as fragments, analogs and derivatives thereof. The polypeptides of the present invention are of human origin.

In accordance with another aspect of the present invention, there are provided polynucleotides (DNA or RNA) which encode such polypeptides.

In accordance with a further aspect of the present invention, there is provided a process for producing such polypeptides by recombinant techniques.

In accordance with yet a further aspect of the present invention, there are provided antibodies against such polypeptides.

In accordance with another embodiment, there is provided a process for using the receptor to screen for receptor antagonists and/or agonists and/or receptor ligands.

In accordance with still another embodiment of the present invention there is provided a process of using such agonists for therapeutic purposes, for example, to treat hypercalcemia, disorders of bone remodeling including osteoporosis, Paget's disease and some forms of hypercalcemia of malignancy.

In accordance with another aspect of the present invention there is provided a process of using such antagonists for treating hypocalcemia.

These and other aspects of the present invention should be apparent to those skilled in the art from the teachings herein.

The following drawings are illustrative of embodiments of the invention and are not meant to limit the scope of the invention as encompassed by the claims.

Figure 1 shows the cDNA sequence and the corresponding deduced amino acid sequence of the G-protein coupled receptor of the present invention. The initial 32 amino acids (highlighted) represent the putative leader sequence. The standard one-letter abbreviation for amino acids is used.

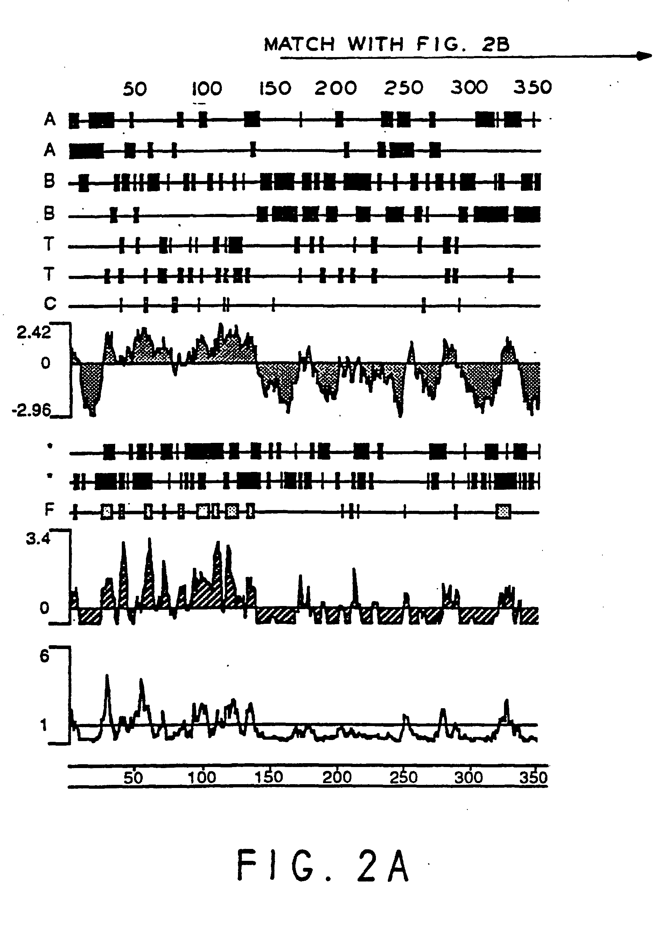

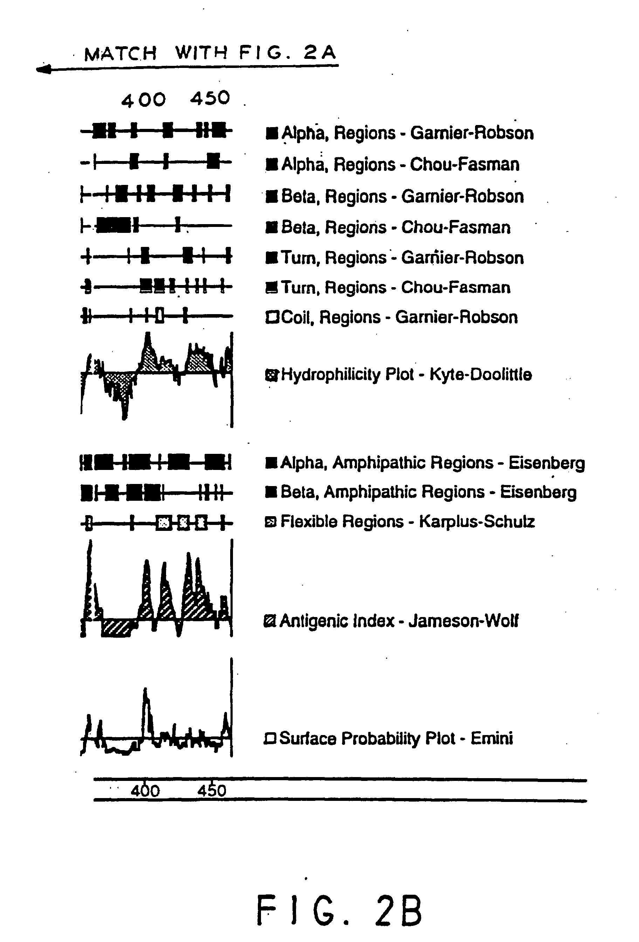

Figure 2 is an illustration of the secondary structural features of the G-protein coupled receptor. The first 7 illustrations set forth the regions of the amino acid sequence which are alpha helices, beta sheets, turn regions or coiled regions. The boxed areas are the areas which correspond to the region indicated. The second set of figures illustrate areas of the amino acid sequence which are exposed to intracellular, cytoplasmic or are membrane-spanning. The hydrophilicity part illustrates areas of the protein sequence which are the lipid bilayer of the membrane and are, therefore, hydrophobic, and areas outside the lipid bilayer membrane which are hydrophilic. The antigenic index corresponds to the hydrophilicity plot, since antigenic areas are areas outside the lipid bilayer membrane and are capable of binding antigens. The surface probability plot further corresponds to the antigenic index and the hydrophilicity plot. The amphipathic plots show those regions of the protein sequences which are polar and non-polar. The flexible regions correspond to the second set of illustrations in the sense that flexible regions are those which are outside the membrane and inflexible regions are transmembrane regions.

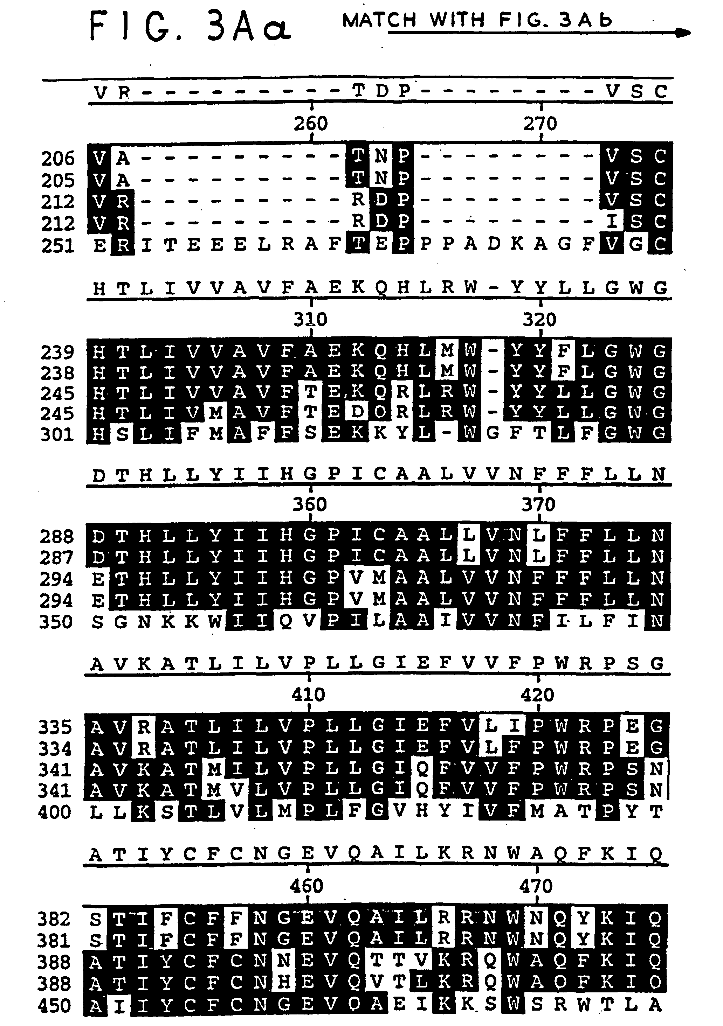

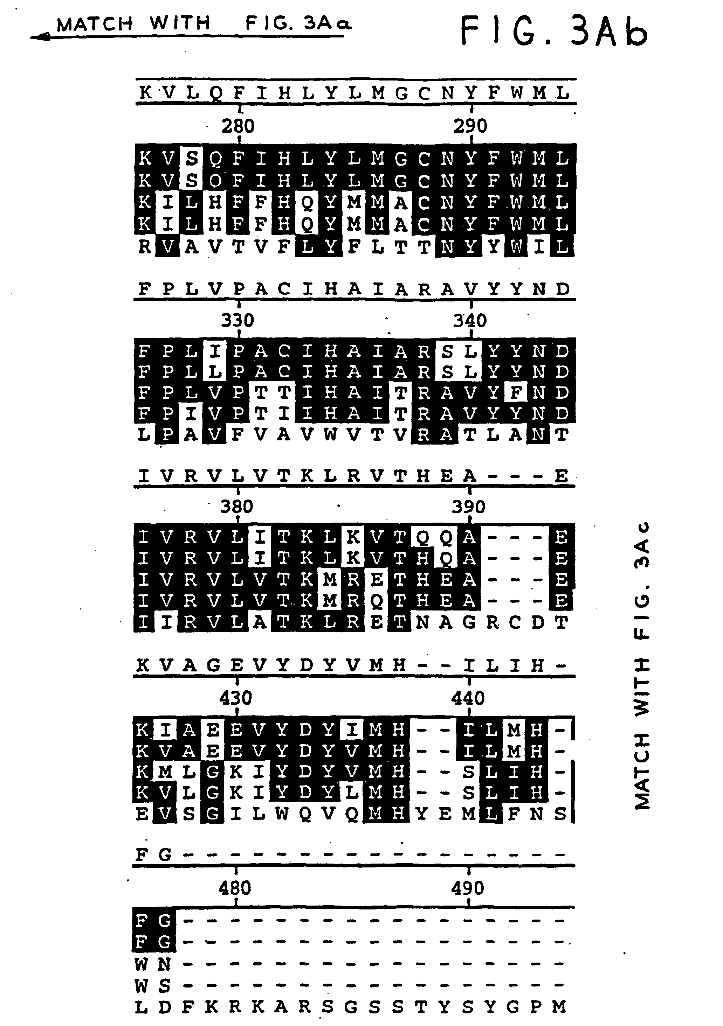

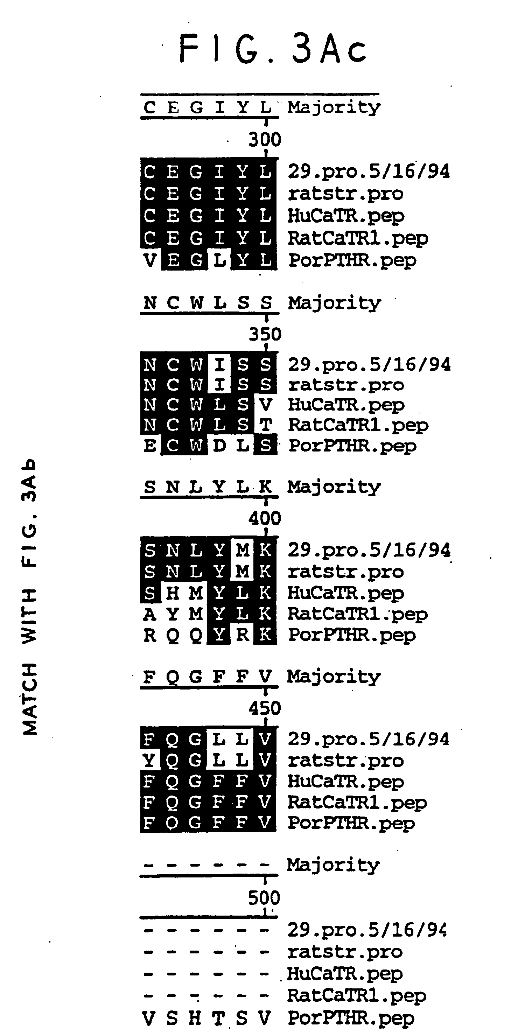

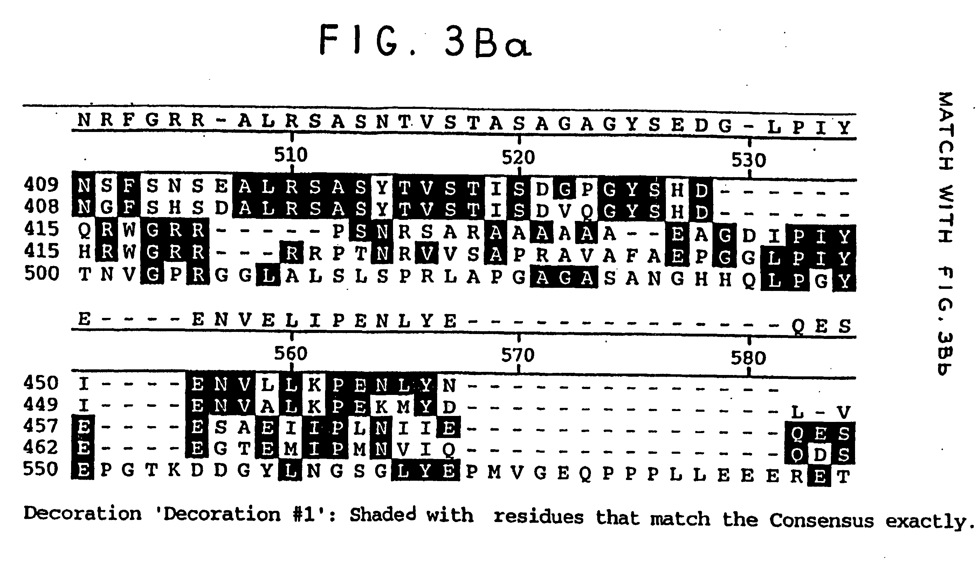

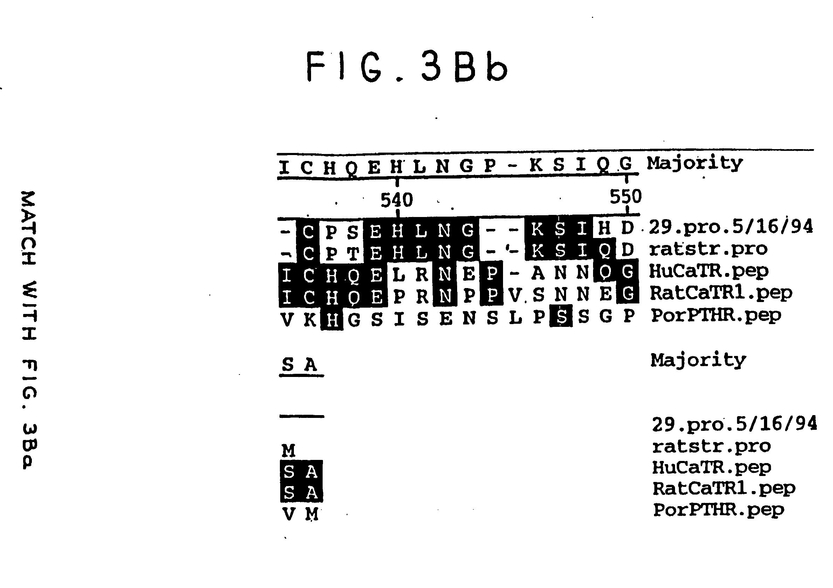

Figure 3 illustrates an amino acid alignment of the G-protein coupled receptor of the present invention and calcitonin receptors from various species of animals. Faded areas are those areas which match with the other amino acid sequences in the figure.

It should be pointed out that sequencing inaccuracies are a common problem which occurs in polynucleotide sequences. Accordingly, the sequence of the drawing is based on several sequencing runs and the sequencing accuracy is considered to be at least 97%.

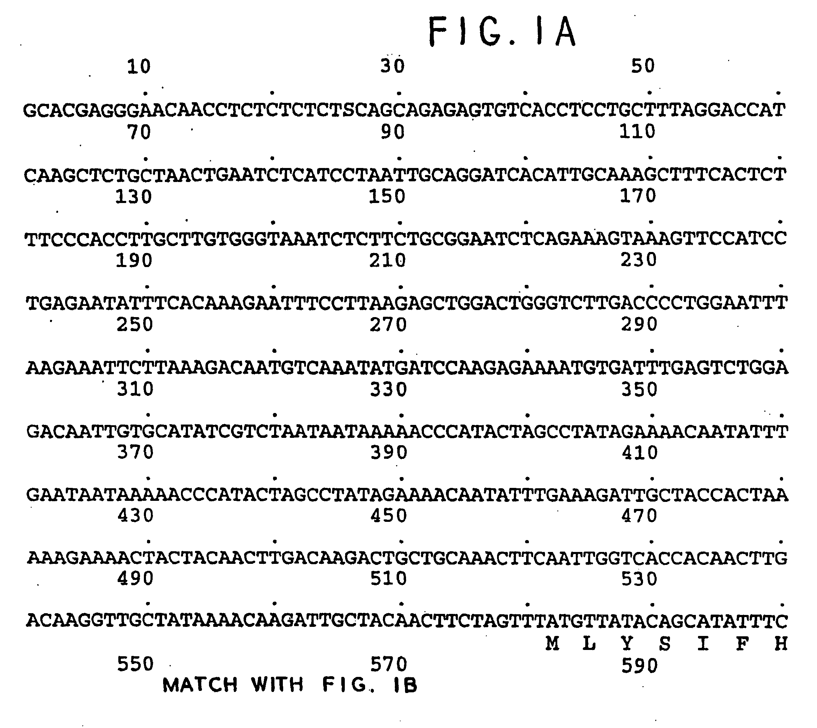

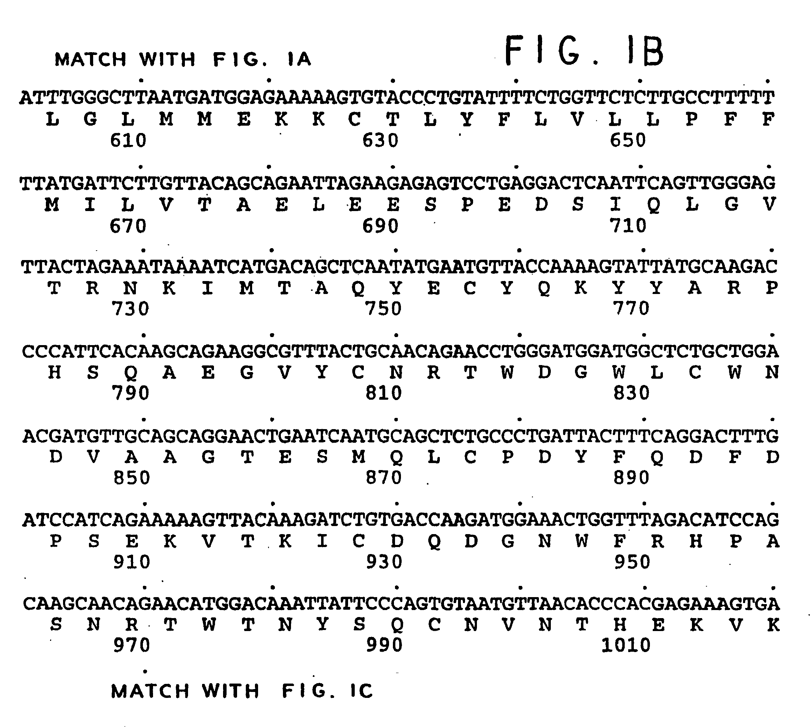

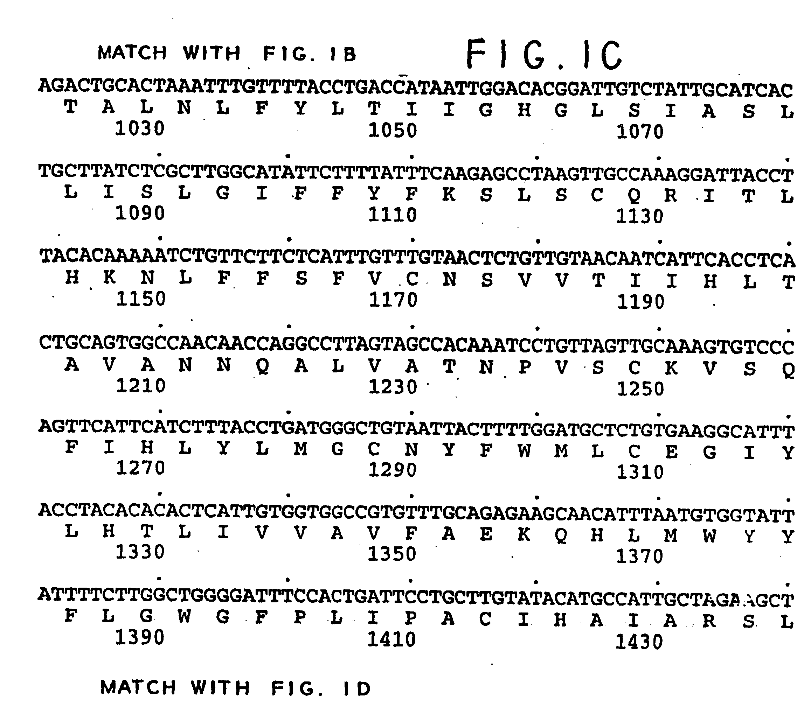

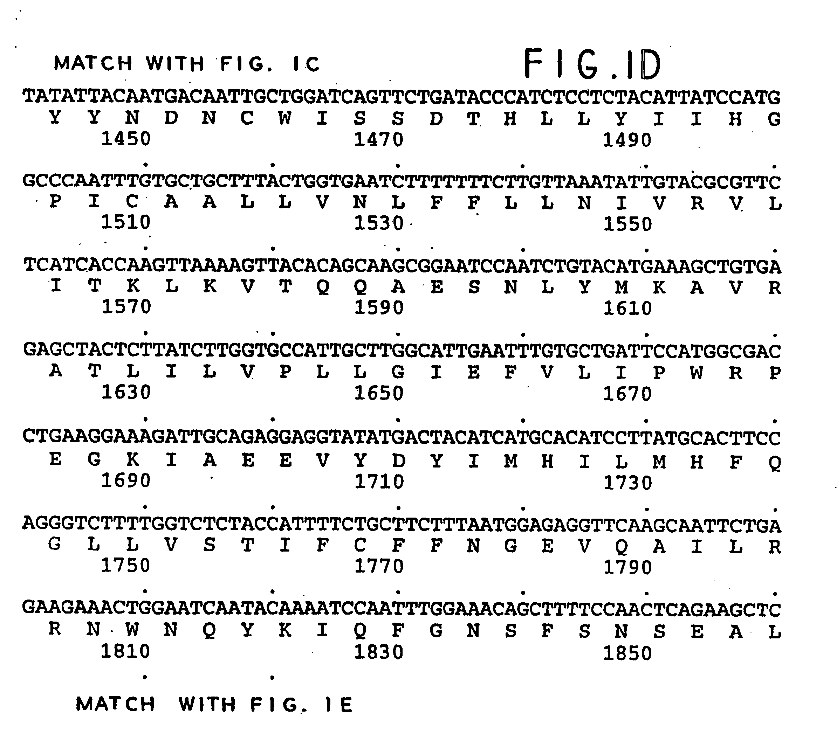

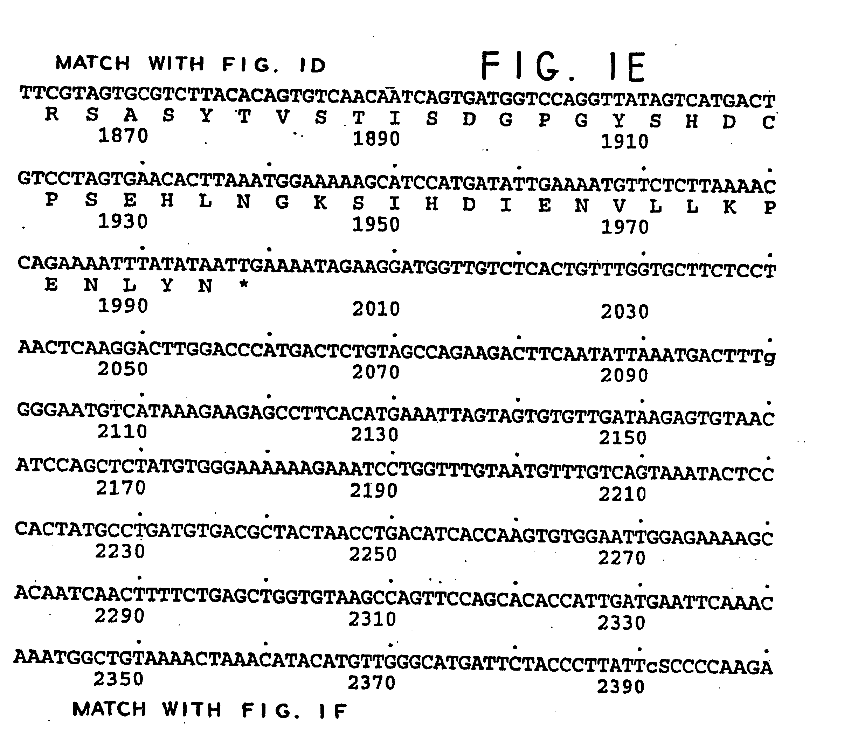

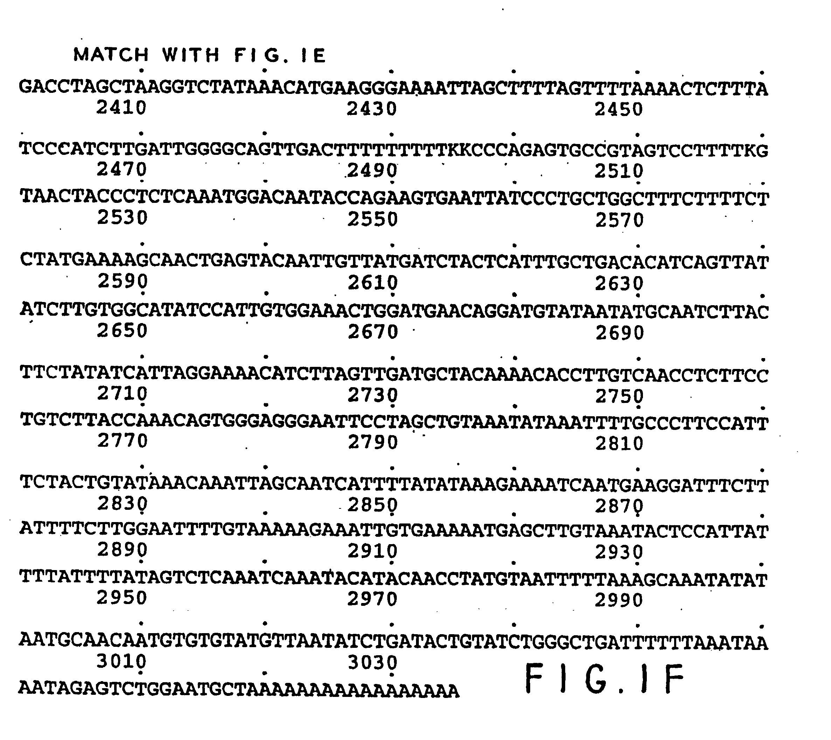

In accordance with an aspect of the present invention, there is provided an isolated nucleic acid (polynucleotide) which encodes for the mature polypeptide having the deduced amino acid sequence of Figure 1 or for the mature polypeptide encoded by the cDNA of the clone deposited as ATCC Deposit No. 75824 on June 24, 1994.

A polynucleotide encoding a polypeptide of the present invention may be found in lung, heart and kidney. The polynucleotide of this invention was discovered in a cDNA library derived from human synovium. It is structurally related to the G protein-coupled receptor family. It contains an open reading frame encoding a protein of 472 amino acid residues of which approximately the first 32 amino acids residues are the putative leader sequence such that the mature protein comprises 440 amino acids. The protein exhibits the highest degree of homology to a human calcitonin receptor with 54 % identity and 72 % similarity over a 469 amino acid stretch.

The polynucleotide of the present invention may be in the form of RNA or in the form of DNA, which DNA includes cDNA, genomic DNA, and synthetic DNA. The DNA may be doublestranded or single-stranded, and if single stranded may be the coding strand or non-coding (anti-sense) strand. The coding sequence which encodes the mature polypeptide may be identical to the coding sequence shown in Figure 1 or that of the deposited clone or may be a different coding sequence which coding sequence, as a result of the redundancy or degeneracy of the genetic code, encodes the same mature polypeptide as the DNA of Figure 1 or the deposited cDNA.

The polynucleotide which encodes for the mature polypeptide of Figure 1 or for the mature polypeptide encoded by the deposited cDNA may include: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 or the polypeptide encoded by the cDNA of the deposited clone. The variant of the polynucleotide may be a naturally occurring allelic variant of the polynucleotide or a non-naturally occurring variant of the polynucleotide.

Thus, the present invention includes polynucleotides encoding the same mature polypeptide as shown in Figure 1 or the same mature polypeptide encoded by the cDNA of the deposited clone as well as variants of such polynucleotides which variants encode for a fragment, derivative or analog of the polypeptide of Figure 1 or the polypeptide encoded by the cDNA of the deposited clone. Such nucleotide variants include deletion variants, substitution variants and addition or insertion variants.

As hereinabove indicated, the polynucleotide may have a coding sequence which is a naturally occurring allelic variant of the coding sequence shown in Figure 1 or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotide of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexa-histidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides . As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which either retain substantially the same biological function or activity as the mature polypeptide encoded by the cDNA of Figure 1 or the deposited cDNA, i.e. function as a G-protein coupled receptor or retain the ability to bind the ligand for the receptor even though the polypeptide does not function as a G-protein coupled receptor, for example, a soluble form of the receptor.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to a G-protein coupled receptor polypeptide which has the deduced amino acid sequence of Figure 1 or which has the amino acid sequence encoded by the deposited cDNA, as well as fragments, analogs and derivatives of such polypeptide.

The terms "fragment," "derivative" and "analog" when referring to the polypeptide of Figure 1 or that encoded by the deposited cDNA, means a polypeptide which either retains substantially the same biological function or activity as such polypeptide, i.e. functions as a G-protein coupled receptor, or retains the ability to bind the ligand or the receptor even though the polypeptide does not function as a G-protein coupled receptor, for example, a soluble form of the receptor. An analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the CRT genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila and Sf9; animal cells such as CHO, COS or Bowes melanoma; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pbs, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation. (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The G-protein coupled receptor polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The G-protein coupled receptor of the present invention may be employed in a process for screening for antagonists and/or agonists for the receptor.

In general, such screening procedures involve providing appropriate cells which express the receptor on the surface thereof. In particular, a polynucleotide encoding the receptor of the present invention is employed to transfect cells to thereby express the G-protein coupled receptor. Such transfection may be accomplished by procedures as hereinabove described.

One such screening procedure involves the use of the melanophores which are transfected to express the G-protein coupled receptor of the present invention. Such a screening technique is described in PCT WO 92/01810 published February 6, 1992.

Thus, for example, such assay may be employed for screening for a receptor antagonist by contacting the melanophore cells which encode the G-protein coupled receptor with both the receptor ligand and a compound to be screened. Inhibition of the signal generated by the ligand indicates that a compound is a potential antagonist for the receptor, i.e., inhibits activation of the receptor.

The screen may be employed for determining an agonist by contacting such cells with compounds to be screened and determining whether such compound generates a signal, i.e., activates the receptor.

Other screening techniques include the use of cells which express the G-protein coupled receptor (for example, transfected CHO cells) in a system which measures extracellular pH changes caused by receptor activation, for example, as described in Science, volume 246, pages 181-296 (October 1989). For example, potential agonists or antagonists may be contacted with a cell which expresses the G-protein coupled receptor and a second messenger response, e.g. signal transduction or pH changes, may be measured to determine whether the potential agonist or antagonist is effective.

Another such screening technique involves introducing RNA encoding the G-protein coupled receptor into xenopus oocytes to transiently express the receptor. The receptor oocytes may then be contacted in the case of antagonist screening with the receptor ligand and a compound to be screened, followed by detection of inhibition of a calcium signal.

Another screening technique involves expressing the G-protein coupled receptor in which the receptor is linked to a phospholipase C or D. As representative examples of such cells, there may be mentioned endothelial cells, smooth muscle cells, embryonic kidney cells, etc. The screening for an antagonist or agonist may be accomplished as hereinabove described by detecting activation of the receptor or inhibition of activation of the receptor from the phospholipase second signal.

Another method involves screening for antagonists by determining inhibition of binding of labeled ligand to cells which have the receptor on the surface thereof. Such a method involves transfecting a eukaryotic cell with DNA encoding the G-protein coupled receptor such that the cell expresses the receptor on its surface and contacting the cell with a potential antagonist in the presence of a labeled form of a known ligand. The ligand can be labeled, e.g., by radioactivity. The amount of labeled ligand bound to the receptors is measured, e.g., by measuring radioactivity of the receptors. If the potential antagonist binds to the receptor as determined by a reduction of labeled ligand which binds to the receptors, the binding of labeled ligand to the receptor is inhibited.

The present invention also provides a method for determining whether a ligand not known to be capable of binding to a G-protein coupled receptor can bind to such receptor which comprises contacting a mammalian cell which expresses a G-protein coupled receptor with the ligand under conditions permitting binding of ligands to the G-protein coupled receptor, detecting the presence of a ligand which binds to the receptor and thereby determining whether the ligand binds to the G-protein coupled receptor. The systems hereinabove described for determining agonists and/or antagonists may also be employed for determining ligands which bind to the receptor.

In general, antagonists for G-protein coupled receptors which are determined by screening procedures may be employed for a variety of therapeutic purposes. For example, such antagonists have been employed for treatment of hypertension, angina pectoris, myocardial infarction, ulcers, asthma, allergies, psychoses, depression, migraine, vomiting, and benign prostatic hypertrophy.

Agonists for G-protein coupled receptors are also useful for therapeutic purposes, such as the treatment of asthma, Parkinson's disease, acute heart failure, hypotension, urinary retention, and osteoporosis.

A potential antagonist is an antibody, or in some cases an oligonucleotide, which binds to the G-protein coupled receptor but does not elicit a second messenger response such that the activity of the G-protein coupled receptor is prevented. Potential antagonists also include proteins which are closely related to the ligand of the G-protein coupled receptor, i.e. a fragment of the ligand, which have lost biological function and when binding to the G-protein coupled receptor, elicit no response.

A potential antagonist also includes an antisense construct prepared through the use of antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of G-protein coupled receptor. The antisense RNA oligonucleotide hybridizes to the mRNA *in vivo* and blocks translation of the mRNA molecule into the G-protein coupled receptor (antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of G-protein coupled receptor.

Another potential antagonist is a small molecule which binds to the G-protein coupled receptor, making it inaccessible to ligands such that normal biological activity is prevented. Examples of small molecules include but are not limited to small peptides or peptide-like molecules.

Potential antagonists also include a soluble form of a G-protein coupled receptor, e.g. a fragment of the receptor, which binds to the ligand and prevents the ligand from interacting with membrane bound G-protein coupled receptors.

The G-protein coupled receptor of the present invention has been putatively identified as a calcitonin receptor. This identification has been made as a result of amino acid sequence homology.

The antagonists may be used to treat hypocalcemia by preventing interaction of calcitonin, a hypocalcemic factor, with the calcitonin receptor. This prevention stops the inhibition of osteoclast-mediated bone resorption. The antagonists may be employed in a composition with a pharmaceutically acceptable carrier, e.g., as hereinafter described.

The agonists identified by the screening method described above, may be employed to treat disorders of bone remodelling, including osteoporosis, Paget's disease of bone and some forms of hypercalcemia of malignancy.

The calcitonin receptor polypeptides and antagonists or agonists which are polypeptides, may be employed in accordance with the present invention by expression of such polypeptides *in vivo*, which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

The calcitonin receptor and antagonists or agonists, may be employed in combination with a suitable pharmaceutical carrier. Such compositions comprise a therapeutically effective amount of the polypeptide, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides of the present invention may be employed in conjunction with other therapeutic compounds.

The pharmaceutical compositions may be administered in a convenient manner such as by the topical, intravenous, intraperitoneal, intramuscular, intranasal or intradermal routes. The pharmaceutical compositions are administered in an amount which is effective for treating and/or prophylaxis of the specific indication. In general, the pharmaceutical compositions will be administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

Antibodies specific to the calcitonin receptor can be employed as a diagnostic tool to locate diseased cells that express calcitonin receptor using any of the methods for such purposes that are available in the art. For example, immunofluorescent or radio active labelling techniques can be performed on tissues or individual cells as a means to identify cells, or to sort cells that express calcitonin receptor. The antibody can also be used to screen bacterial expression libraries for the presence of calcitonin receptor, or for molecules that are related to the calcitonin receptor.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the cDNA is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include in situ hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the EST was derived, and the longer the better. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. et al., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units to T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973).

### Example 1

### Bacterial Expression and Purification of CTR

The DNA sequence encoding CTR, ATCC # 75824, is initially amplified using PCR oligonucleotide primers corresponding to the 5' end sequences of the processed protein (minus the signal peptide sequence) and the vector sequences 3' to the CTR gene. Additional nucleotides corresponding to the CTR coding sequence were added to the 5' and 3' sequences respectively. The 5' oligonucleotide primer has the sequence 5' GACTAAAGCTTAATG TTATACAGCATATTT 3' contains a HindIII restriction enzyme site followed by 18 nucleotides of the CTR coding sequence starting from the presumed terminal amino acid of the processed protein codon. The 3' sequence 5' GAACTTCTAGACCGTCAATT ATATAAATTTTTC 3' contains complementary sequences to an XbaI site and is followed by 18 nucleotides of the CTR coding sequence. The restriction enzyme sites correspond to the restriction enzyme sites on the bacterial expression vector pQE-9 (Qiagen, Inc. 9259 Eton Avenue, Chatsworth, CA, 91311). pQE-9 encodes antibiotic resistance (Amp'), a bacterial origin of replication (ori), an IPTG-regulatable promoter operator (P/O), a ribosome binding site (RBS), a 6-His tag and restriction enzyme sites. pQE-9 was then digested with HindIII and XbaI. The amplified sequences were ligated into pQE-9 and were inserted in frame with the sequence encoding for the histidine tag and the RBS. The ligation mixture was then used to transform E. coli strain available from Qiagen under the trademark M15/rep 4 by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies were selected. Plasmid DNA was isolated and confirmed by restriction analysis. Clones containing the desired constructs were grown overnight (O/N) in liquid culture in LB media supplemented with both Amp (100 ug/ml) and Kan (25 ug/ml). The O/N culture is used to inoculate a large culture at a ratio of 1:100 to 1:250. The cells were grown to an optical density 600 (O.D.⁶⁰⁰) of between 0.4 and 0.6. IPTG ("Isopropyl-B-D-thiogalacto pyranoside") was then added to a final concentration of 1 mM. IPTG induces by inactivating the lacI repressor, clearing the P/O leading to increased gene expression. Cells were grown an extra 3 to 4 hours. Cells were then harvested by centrifugation. The cell pellet was solubilized in the chaotropic agent 6 Molar Guanidine HCl. After clarification, solubilized was purified from this solution by chromatography on a Nickel-Chelate column under conditions that allow for tight binding by proteins containing the 6-His tag. Hochuli, E. et al., J. Chromatography 411:177-184 (1984). The CTR was eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized). After incubation in this solution for 12 hours the protein was dialyzed to 10 mmolar sodium phosphate.

### Example 2

### Expression of Recombinant CTR in COS cells

The expression of plasmid, CTR HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire CTR precursor and a HA tag fused in frame to its 3' end was cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37, 767). The infusion of HA tag to our target protein allows easy detection of the recombinant protein with an antibody that recognizes the HA epitope.

The plasmid construction strategy is described as follows:

The DNA sequence encoding the CTR, ATCC # 75824, was constructed by PCR on the original EST cloned using two primers: the 5' primer 5' GTCCGAAGCTTGCCAC CATGTTATACAGCATATTT 3' contains a HindIII site followed by 18 nucleotides of CTR coding sequence starting from the initiation codon; the 3' sequence 5' CTAGCTCGAGTCAAGCGT AGTCTGGGACCTCGTATGGGTAGCAATTATATAAATTTTCTGG 3' contains complementary sequences to an XhoI site, translation stop codon, HA tag and the last 18 nucleotides of the CTR coding sequence (not including the stop codon). Therefore, the PCR product contains a HindIII site, CTR coding sequence followed by HA tag fused in frame, a translation termination stop codon next to the HA tag, and an XhoI site. The PCR amplified DNA fragment and the vector, pcDNAI/Amp, were digested with HindIII and XhoI restriction enzyme and ligated. The ligation mixture was transformed into E. coli strain SURE (available from Stratagene Cloning Systems, 11099 North Torrey Pines Road, La Jolla, CA 92037) the transformed culture was plated on ampicillin media plates and resistant colonies were selected. Plasmid DNA was isolated from transformants and examined by restriction analysis for the presence of the correct fragment. For expression of the recombinant CTR, COS cells were transfected with the expression vector by DEAE-DEXTRAN method. (J. Sambrook, E. Fritsch, T. Maniatis, Molecular Cloning: A Laboratory Manual, Cold Spring Laboratory Press, (1989)). The expression of the CTR HA protein was detected by radiolabelling and immunoprecipitation method. (E. Harlow, D. Lane, Antibodies: A Laboratory Manual, Cold Spring Harbor Laboratory Press, (1988)). Cells were labelled for 8 hours with ³⁵S-cysteine two days post transfection. Culture media were then collected and cells were lysed with detergent (RIPA buffer (150 mM NaCl, 1% NP-40, 0.1% SDS, 1% NP-40, 0.5% DOC, 50mM Tris, pH 7.5). (Wilson, I. et al., Id. 37:767 (1984)). Both cell lysate and culture media were precipitated with a HA specific monoclonal antibody. Proteins precipitated were analyzed on 15% SDS-PAGE gels.

### Example 3

### Cloning and expression of CTR using the baculovirus expression system

The DNA sequence encoding the full length CTR protein, ATCC # 75824, was amplified using PCR oligonucleotide primers corresponding to the 5' and 3' sequences of the gene:

The 5' primer has the sequence 5' GTCC**GGATCC**GCCACCATGTTATACAGCATATTT 3' and contains a BamHI restriction enzyme site (in bold) followed by 6 nucleotides resembling an efficient signal for the initiation of translation in eukaryotic cells (J. Mol. Biol. 1987, 196, 947-950, Kozak, M.), and is just behind the first 18 nucleotides of the CTR gene (the initiation codon for translation "ATG" is underlined).

The 3' primer has the sequence 5' GTCCGGATCCGCCACCATGTTATACAGCATATTT 3' and contains the cleavage site for the restriction endonuclease BamHI and 18 nucleotides complementary to the 3' non-translated sequence of the CTR gene. The amplified sequences were isolated from a 1% agarose gel using a commercially available kit ("Geneclean," BIO 101 Inc., La Jolla, Ca.). The fragment was then digested with the endonuclease BamHI and then purified on a 1% agarose gel. This fragment is designated F2.

The vector pRG1 (modification of pVL941 vector, discussed below) is used for the expression of the CTR protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhedrosis virus (AcMNPV) followed by the recognition site for the restriction endonuclease BamHI. The polyadenylation site of the simian virus (SV)40 is used for efficient polyadenylation. For an easy selection of recombinant viruses the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of cotransfected wild-type viral DNA. Many other baculovirus vectors could be used in place of pRG1 such as pAc373, pVL941 and pAcIM1 (Luckow, V.A. and Summers, M.D., Virology, 170:31-39).

The plasmid was digested with the restriction enzyme BamHI and then dephosphorylated using calf intestinal phosphatase by procedures known in the art. The DNA was then isolated and purified on a 1% agarose gel as described above. This vector DNA is designated V2.

Fragment F2 and the dephosphorylated plasmid V2 were ligated with T4 DNA ligase. E.coli HB101 cells were then transformed and bacteria identified that contained the plasmid (pBacCTR) with the CTR gene using the enzyme BamHI. The sequence of the cloned fragment was confirmed by DNA sequencing.

5 µg of the plasmid pBacCTR were cotransfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBacCTR were mixed in a sterile well of a microtiter plate containing 50 µl of serum free Grace's medium (Life Technologies Inc., Gaithersburg, MD). Afterwards 10 µl Lipofectin plus 90 µl Grace's medium were added, mixed and incubated for 15 minutes at room temperature. Then the transfection mixture was added dropwise to the Sf9 insect cells (ATCC CRL 1711) seeded in a 35 mm tissue culture plate with 1ml Grace' medium without serum. The plate was rocked back and forth to mix the newly added solution. The plate was then incubated for 5 hours at 27°C. After 5 hours the transfection solution was removed from the plate and 1 ml of Grace's insect medium supplemented with 10% fetal calf serum was added. The plate was put back into an incubator and cultivation continued at 27°C for four days.

After four days the supernatant was collected and a plaque assay performed similar as described by Summers and Smith (supra). As a modification an agarose gel with "Blue Gal" (Life Technologies Inc., Gaithersburg) was used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution of the viruses was added to the cells, blue stained plaques were picked with the tip of an Eppendorf pipette. The agar containing the recombinant viruses was then resuspended in an Eppendorf tube containing 200 µl of Grace's medium. The agar was removed by a brief centrifugation and the supernatant containing the recombinant baculoviruses was used to infect Sf9 cells seeded in 35 mm dishes. Four days later the supernatants of these culture dishes were harvested and then stored at 4°C.

Sf9 cells were grown in Grace's medium supplemented with 10% heat-inactivated FBS. The cells were infected with the recombinant baculovirus V-CTR at a multiplicity of infection (MOI) of 2. Six hours later the medium was removed and replaced with SF900 II medium minus methionine and cysteine (Life Technologies Inc., Gaithersburg). 42 hours later 5 µCi of ³⁵S-methionine and 5 µCi ³⁵S cysteine (Amersham) were added. The cells were further incubated for 16 hours before they were harvested by centrifugation and the labelled proteins visualized by SDS-PAGE and autoradiography.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in Figure 1;
(b) polynucleotides having the coding sequence as shown in Figure 1 encoding at least the mature form of the polypeptide;
(c) polynucleotides encoding the polypeptide having the amino acid sequence of at least the mature form of the polypeptide encoded by the cDNA contained in ATCC 75824;
(d) polynucleotides having the coding sequence of the cDNA contained in ATCC 75824 encoding at least the mature form of the polypeptide;
(e) polynucleotides encoding a fragment of a polypeptide encoded by a polynucleotide of any one of (a) to (d) and having calcitonin receptor activity; and
(f) polynucleotides which specifically hybridize to a polynucleotide of any one of (a) to (e), wherein said polynucleotide is at least 95% identical to the polynucleotide of any one of (a) to (e) and which encode a polypeptide having calcitonin receptor activity;
or the complementary strand of such a polynucleotide.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the polynucleotide of any one of claims 1 to 4 or the vector of claim 5 or 6.

8. A process for producing a polypeptide encoded by the polynucleotide of any one of claims 1 to 4 or soluble fragment thereof comprising culturing the host cell of claim 7 and recovering the polypeptide encoded by said polynucleotide from the culture.

9. A process for producing cells capable of expressing a polypeptide encoded by the polynucleotide of any one of claims 1 to 4 or soluble fragment thereof comprising genetically engineering cells with the vector of claim 5 or 6.

10. A polypeptide having the amino acid sequence encoded by a polynucleotide of any one of claims 1 to 4 or obtainable by the process of claim 8.

11. An antibody against the polypeptide of claim 10.

12. A pharmaceutical composition comprising the polynucleotide of any one of claims 1 to 4, the polypeptide of claim 10 or the antibody of claim 11 and optionally a pharmaceutically acceptable carrier.

13. A diagnostic composition comprising the polynucleotide of any one of claims 1 to 4 or the antibody of claim 11.

14. A process for identifying antagonists and agonists to the polypeptide of claim 10 comprising:
(a) expressing the G-protein coupled receptor of claim 10 on the surface of a cell;
(b) contacting the cell with a receptor ligand and compound to be screened;
(c) determining whether a second signal is generated from the interaction of the ligand and the receptor; and
(d) identifying if the compound to be screened is an agonist or antagonist.

15. A process for determining whether a ligand not known to be capable of binding to a the polypeptide of claim 10 can bind thereto comprising:
(a) contacting a mammalian cell which expresses the G-protein coupled receptor of claim 10 with a potential ligand;
(b) detecting the presence of the ligand which binds to the receptor; and
(c) determining whether the ligand binds to the G-protein coupled receptor.

## Patentansprüche

1. Polynucleotid ausgewählt aus der Gruppe bestehend aus
(a) Polynucleotiden, die zumindest die reife Form des Polypeptids codieren, das die abgeleitete Aminosäuresequenz wie in Figur 1 gezeigt aufweist;
(b) Polynucleotiden, die die codierende Sequenz wie in Figur 1 gezeigt aufweisen, die zumindest die reife Form des Polypetids codiert;
(c) Polynucleotiden, die das Polypeptid codieren, das die Aminosäuresequenz von zumindest der reifen Form des Polypeptids aufweist, das von der in ATCC 75824 enthaltenen cDNA codiert wird;
(d) Polynucleotiden, die die codierende Sequenz der in ATCC 75824 enthaltenen cDNA aufweisen, die zumindest die reife Form des Polypetids codiert;
(e) Polynucleotiden, die ein Fragment eines Polypeptids codieren, das von einem Polynucleotid gemäß einem der Teile (a) bis (d) codiert wird, wobei das Fragment die Aktivität eines Calcitonin-Rezeptors aufweist;
(f) Polynucleotiden, die spezifisch mit einem gemäß einem der Teile (a) bis (e) definierten Polynucleotid hybridisieren, wobei das Polynucleotid zumindest zu 95% identisch ist mit dem Polynucleotid gemäß einem der Teile (a) bis (d), und die ein Polypeptid codieren, das die Aktivität eines Calcitonin-Rezeptors aufweist;
oder der komplementäre Strang eines solchen Polynucleotids.

2. Polynucleotid nach Anspruch 1, das DNA ist.

3. DNA nach Anspruch 2, die genomische DNA ist.

4. Polynucleotid nach Anspruch 1, das RNA ist.

5. Vektor, enthaltend das Polynucleotid nach einem der Ansprüche 1 bis 4.

6. Vektor nach Anspruch 5, wobei das Polynucleotid funktionell verbunden ist mit Expressionskontrollsequenzen, die eine Expression in prokaryontischen oder eukaryontischen Wirtszellen erlauben.

7. Wirtszelle, die genetisch verändert ist mit dem Polynucleotid nach einem der Ansprüche 1 bis 4 oder dem Vektor nach Anspruch 5 oder 6.

8. Verfahren zur Herstellung eines Polypeptids, das von einem Polynucleotid nach einem der Ansprüche 1 bis 4 codiert wird, oder eines löslichen Fragments davon, umfassend das Züchten der Wirtszelle nach Anspruch 7 und Gewinnen des Polypeptids, das von dem Polynucleotid codiert wird, aus der Kultur.

9. Verfahren zur Herstellung von Zellen, die in der Lage sind, ein Polypeptid zu exprimieren, das von einem Polynucleotid nach einem der Ansprüche 1 bis 4 codiert wird, oder ein lösliches Fragment davon, umfassend das genetische Verändern von Zellen mit dem Vektor nach Anspruch 5 oder 6.

10. Polypeptid mit der Aminosäuresequenz, die von einem Polynucleotid nach einem der Ansprüche 1 bis 4 codiert wird, oder erhältlich durch das Verfahren nach Anspruch 8.

11. Antikörper gegen das Polypeptid nach Anspruch 10.

12. Arzneimittel, umfassend das Polynucleotid nach einem der Ansprüche 1 bis 4, das Polypeptid nach Anspruch 10 oder den Antikörper nach Anspruch 11 und gegebenenfalls einen pharmazeutisch verträglichen Träger.

13. Diagnostische Zusammensetzung, umfassend ein Polynucleotid nach einem der Ansprüche 1 bis 4 oder den Antikörper nach Anspruch 11.

14. Verfahren zum Auffinden eines Antagonisten oder Agonisten des Polypeptides nach Anspruch 10, umfassend:
(a) Expression des G-Protein-gekoppelten Rezeptors nach Anspruch 10 an der Oberfläche einer Zelle;
(b) Inkontaktbringen der Zelle mit einem Liganden des Rezeptors und einer Verbindung, die bestimmt werden soll;
(c) Bestimmen, ob die Wechselwirkung des Liganden mit dem Rezeptor ein zweites Signal erzeugt; und
(d) Auffinden, ob die Verbindung, die bestimmt werden soll, ein Agonist oder Antagonist ist.

15. Verfahren zur Bestimmung, ob ein Ligand, von dem nicht bekannt ist, ob er das Polypeptid nach Anspruch 10 binden kann, an dieses bindet, umfassend:
(a) Inkontaktbringen einer Säugerzelle, die den G-Protein-gekoppelten Rezeptor nach Anspruch 10 exprimiert, mit einem potentiellen Liganden;
(b) Nachweisen der Anwesenheit des Liganden, der an den Rezeptor bindet; und
(c) Bestimmen, ob der Ligand an den G-Protein-gekoppelten Rezeptor bindet.

## Revendications

1. Polynucléotide choisi au sein du groupe composé des :
(a) polynucléotides codant au moins la forme mature du polypeptide possédant la séquence d'acides aminés déduite telle que représentée à la figure 1 ;
(b) des polynucléotides possédant la séquence codante telle que représentée à la figure 1 codant au moins la forme mature du polypeptide ;
(c) des polynucléotides codant le polypeptide possédant la séquence d'acides aminés d'au moins la forme mature du polypeptide codé par l'ADNc contenu dans ATCC 75824 ;
(d) des polynucléotides possédant la séquence codante de l'ADNc contenu dans ATCC 75824 codant au moins la forme mature du polypeptide ;
(e) des polynucléotides codant un fragment d'un polypeptide selon l'un quelconque des points (a) à (d) et possédant l'activité de récepteur de la calcitonine ; et
(f) des polynucléotides qui s'hybrident spécifiquement avec un polynucléotide selon l'un quelconque des points (a) à (e), dans lesquels ledit polynucléotide présente un degré d'identité d'au moins 95% avec le polynucléotide de l'un quelconque des points (a) à (e) et qui code un polypeptide possédant l'activité du récepteur de la calcitonine ;
ou le brin complémentaire d'un tel polynucléotide.

2. Polynucléotide de la revendication 1 qui est de l'ADN.

3. ADN de la revendication 2 qui est de l'ADN génomique.

4. Polynucléotide de la revendication 1 qui est de l'ARN.

5. Vecteur contenant le polynucléotide de l'une quelconque des revendications 1 à 4.

6. Vecteur selon la revendication 5 dans lequel le polynucléotide est lié de manière opérationnelle à des séquences de régulation de l'expression permettant l'expression dans des cellules hôtes procaryotes ou eucaryotes.

7. Cellule hôte génétiquement modifiée avec le polynucléotide de l'une quelconque des revendications 1 à 4 ou avec le vecteur des revendications 5 ou 6.

8. Procédé de préparation d'un polypeptide codé par le polynucléotide de l'une quelconque des revendications 1 à 4 ou un fragment soluble de celui-ci comprenant la culture de la cellule hôte de la revendication 7 et la récupération du polypeptide codé par ledit polynucléotide à partir de la culture.

9. Procédé de production de cellules capables d'exprimer un polypeptide codé par le polynucléotide selon l'une quelconque des revendications 1 à 4 ou un fragment soluble de celui-ci comprenant la modification génétique des cellules avec le vecteur de la revendication 5 ou 6.

10. Polypeptide possédant la séquence d'acides aminés codée par un polynucléotide selon l'une quelconque des revendications 1 à 4 ou pouvant être obtenu avec le procédé de la revendication 8.

11. Anticorps dirigé contre le polypeptide de la revendication 10.

12. Composition pharmaceutique comprenant le polynucléotide de l'une quelconque des revendications 1 à 4, le polypeptide de la revendication 10 ou l'anticorps de la revendication 11 et éventuellement un support pharmaceutiquement acceptable.

13. Composition de diagnostic comprenant le polynucléotide de l'une quelconque des revendications 1 à 4 ou l'anticorps de la revendication 11.

14. Procédé destiné à identifier des antagonistes et des agonistes du polypeptide de la revendication 10 comprenant les étapes consistant à:
(a) exprimer le récepteur couplé à la protéine G de la revendication 10 à la surface d'une cellule;
(b) mettre en contact la cellule avec un ligand du récepteur et un composé à cribler ;
(c) déterminer si un second signal est généré à partir de l'interaction du ligand et du récepteur ; et
(d) identifier si le composé à cribler est un agoniste ou un antagoniste.

15. Procédé destiné à déterminer si un ligand qui n'est pas connu comme étant capable de se lier au polypeptide de la revendication 10 est susceptible de se lier à celui-ci comprenant les étapes consistant à:
(a) mettre en contact une cellule de mammifère qui exprime le récepteur couplé à la protéine G de la revendication 10 avec un ligand potentiel ;
(b) détecter la présence du ligand qui se lie au récepteur ; et
(c) déterminer si le ligand se lie au récepteur couplé à la protéine G.


### SEQUENCE LISTING

(1) GENERAL INFORMATION:
   (i) APPLICANT: LI, ET AL.
   (ii) TITLE OF INVENTION: Calcitonin Receptor
   (iii) NUMBER OF SEQUENCES: 2
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: Concurrently
      (C) CLASSIFICATION:
   (vii) PRIOR APPLICATION DATA
      (A) APPLICATION NUMBER:
      (B) FILING DATE:
   (viii) ATTORNEY/AGENT INFORMATION:
      (A) NAME: FERRARO, GREGORY D.
      (B) REGISTRATION NUMBER: 36,134
      (C) REFERENCE/DOCKET NUMBER: 325800-193
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 3035 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   GCACGAGGGA ACAACCTCTC TCTCTSCAGC AGAGAGTGTC ACCTCCTGCT TTAGGACCAT    60
   CAAGCTCTGC TAACTGAATC TCATCCTAAT TGCAGGATCA CATTGCAAAG CTTTCACTCT   120
   TTCCCACCTT GCTTGTGGGT AAATCTCTTC TGCGGAATCT CAGAAAGTAA AGTTCCATCC   180
   TGAGAATATT TCACAAAGAA TTTCCTTAAG AGCTGGACTG GGTCTTGACC CCTGGAATTT   240
   AAGAAATTCT TAAAGACAAT GTCAAATATG ATCCAAGAGA AAATGTGATT TGAGTCTGGA   300
   GACAATTGTG CATATCGTCT AATAATAAAA ACCCATACTA GCCTATAGAA AACAATATTT   360
   GAATAATAAA AACCCATACT AGCCTATAGA AAACAATATT TGAAAGATTG CTACCACTAA   420
   AAAGAAAACT ACTACAACTT GACAAGACTG CTGCAAACTT CAATTGGTCA CCACAACTTG   480
   ACAAGGTTGC TATAAAACAA GATTGCTACA ACTTCTAGTT TATGTTATAC AGCATATTTC   540
   ATTTGGGCTT AATGATGGAG AAAAAGTGTA CCCTGTATTT TCTGGTTCTC TTGCCTTTTT   600
   TTATGATTCT TGTTACAGCA GAATTAGAAG AGAGTCCTGA GGACTCAATT CAGTTGGGAG   660
   TTACTAGAAA TAAAATCATG ACAGCTCAAT ATGAATGTTA CCAAAAGTAT TATGCAAGAC   720
   CCCATTCACA AGCAGAAGGC GTTTACTGCA ACAGAACCTG GGATGGATGG CTCTGCTGGA   780
   ACGATGTTGC AGCAGGAACT GAATCAATGC AGCTCTGCCC TGATTACTTT CAGGACTTTG   840
   ATCCATCAGA AAAAGTTACA AAGATCTGTG ACCAAGATGG AAACTGGTTT AGACATCCAG   900
   CAAGCAACAG AACATGGACA AATTATTCCC AGTGTAATGT TAACACCCAC GAGAAAGTGA   960
   AGACTGCACT AAATTTGTTT TACCTGACCA TAATTGGACA CGGATTGTCT ATTGCATCAC  1020
   TGCTTATCTC GCTTGGCATA TTCTTTTATT TCAAGAGCCT AAGTTGCCAA AGGATTACCT 1080
   TACACAAAAA TCTGTTCTTC TCATTTGTTT GTAACTCTGT TGTAACAATC ATTCACCTCA 1140
   CTGCAGTGGC CAACAACCAG GCCTTAGTAG CCACAAATCC TGTTAGTTGC AAAGTGTCCC 1200
   AGTTCATTCA TCTTTACCTG ATGGGCTGTA ATTACTTTTG GATGCTCTGT GAAGGCATTT 1260
   ACCTACACAC ACTCATTGTG GTGGCCGTGT TTGCAGAGAA GCAACATTTA ATGTGGTATT 1320
   ATTTTCTTGG CTGGGGATTT CCACTGATTC CTGCTTGTAT ACATGCCATT GCTAGAAGCT 1380
   TATATTACAA TGACAATTGC TGGATCAGTT CTGATACCCA TCTCCTCTAC ATTATCCATG 1440
   GCCCAATTTG TGCTGCTTTA CTGGTGAATC TTTTTTTCTT GTTAAATATT GTACGCGTTC 1500
   TCATCACCAA GTTAAAAGTT ACACAGCAAG CGGAATCCAA TCTGTACATG AAAGCTGTGA 1560
   GAGCTACTCT TATCTTGGTG CCATTGCTTG GCATTGAATT TGTGCTGATT CCATGGCGAC 1620
   CTGAAGGAAA GATTGCAGAG GAGGTATATG ACTACATCAT GCACATCCTT ATGCACTTCC 1680
   AGGGTCTTTT GGTCTCTACC ATTTTCTGCT TCTTTAATGG AGAGGTTCAA GCAATTCTGA 1740
   GAAGAAACTG GAATCAATAC AAAATCCAAT TTGGAAACAG CTTTTCCAAC TCAGAAGCTC 1800
   TTCGTAGTGC GTCTTACACA GTGTCAACAA TCAGTGATGG TCCAGGTTAT AGTCATGACT 1860
   GTCCTAGTGA ACACTTAAAT GGAAAAAGCA TCCATGATAT TGAAAATGTT CTCTTAAAAC 1920
   CAGAAAATTT ATATAATTGA AAATAGAAGG ATGGTTGTCT CACTGTTTGG TGCTTCTCCT 1980
   AACTCAAGGA CTTGGACCCA TGACTCTGTA GCCAGAAGAC TTCAATATTA AATGACTTTG 2040
   GGGAATGTCA TAAAGAAGAG CCTTCACATG AAATTAGTAG TGTGTTGATA AGAGTGTAAC 2100
   ATCCAGCTCT ATGTGGGAAA AAAGAAATCC TGGTTTGTAA TGTTTGTCAG TAAATACTCC 2160
   CACTATGCCT GATGTGACGC TACTAACCTG ACATCACCAA GTGTGGAATT GGAGAAAAGC 2220
   ACAATCAACT TTTCTGAGCT GGTGTAAGCC AGTTCCAGCA CACCATTGAT GAATTCAAAC 2280
   AAATGGCTGT AAAACTAAAC ATACATGTTG GGCATGATTC TACCCTTATT CSCCCCAAGA 2340
   GACCTAGCTA AGGTCTATAA ACATGAAGGG AAAATTAGCT TTTAGTTTTA AAACTCTTTA 2400
   TCCCATCTTG ATTGGGGCAG TTGACTTTTT TTTTKKCCCA GAGTGCCGTA GTCCTTTTKG 2460
   TAACTACCCT CTCAAATGGA CAATACCAGA AGTGAATTAT CCCTGCTGGC TTTCTTTTCT 2520
   CTATGAAAAG CAACTGAGTA CAATTGTTAT GATCTACTCA TTTGCTGACA CATCAGTTAT 2580
   ATCTTGTGGC ATATCCATTG TGGAAACTGG ATGAACAGGA TGTATAATAT GCAATCTTAC 2640
   TTCTATATCA TTAGGAAAAC ATCTTAGTTG ATGCTACAAA ACACCTTGTC AACCTCTTCC 2700
   TGTCTTACCA AACAGTGGGA GGGAATTCCT AGCTGTAAAT ATAAATTTTG CCCTTCCATT 2760
   TCTACTGTAT AAACAAATTA GCAATCATTT TATATAAAGA AAATCAATGA AGGATTTCTT 2820
   ATTTTCTTGG AATTTTGTAA AAAGAAATTG TGAAAAATGA GCTTGTAAAT ACTCCATTAT 2880
   TTTATTTTAT AGTCTCAAAT CAAATACATA CAACCTATGT AATTTTTAAA GCAAATATAT 2940
   AATGCAACAA TGTGTGTATG TTAATATCTG ATACTGTATC TGGGCTGATT TTTTAAATAA 3000
   AATAGAGTCT GGAATGCTAA AAAAAAAAAA AAAAA                            3035
(2) INFORMATION FOR SEQ ID NO:2:
   (i) SEQUENCE CHARACTERISTICS
      (A) LENGTH: 472 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2: